# EUROPÄISCHE PATENTANMELDUNG

(11) **EP 0 529 749 A1**
(43) Veröffentlichungstag der Anmeldung: **03.03.1993**
(21) Anmeldenummer: 92250203.4
(22) Anmeldetag: 06.08.1992
(51) Int. Cl.: C07D 333/32, C07D 409/06, A61K 31/38, A61K 31/44

(54) **Leukotrien-B4-Antagonisten, Verfahren zu ihrer Herstellung und ihre Verwendung als Arzneimittel**

(30) Priorität: 15.08.1991 DE 4127193
(71) Anmelder: SCHERING AKTIENGESELLSCHAFT, D-13353 Berlin (DE)
(72) Erfinder: Skuballa, Werner, Dr., W-1000 Berlin 28 (DE); Buchmann, Bernd, Dr., W-1000 Berlin 21 (DE); Heindl, Josef, Dr., W-1000 Berlin 38 (DE); Fröhlich, Wolfgang, Dr., W-1000 Berlin 31 (DE); Ekerdt, Roland, Dr., W-1000 Berlin 28 (DE); Giesen, Claudia, Dr., W-1000 Berlin 20 (DE)

(57) **Zusammenfassung**

1) Leukotrien-B₄-Antagonisten der Formel I, 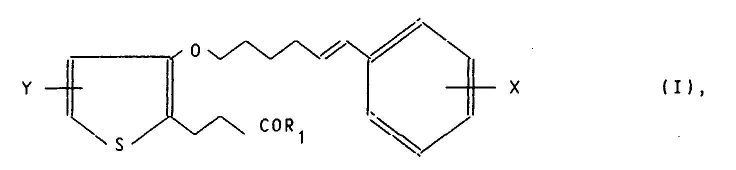
werden beschrieben,
worin
- X: C₁-C₄-Alkoxy oder -S(O)ₚ-(C₁-C₄)-Alkyl,
- p: 0, 1 oder 2
- Y: ein Wasserstoffatom oder den Rest COACOR₂ mit A in der Bedeutung einer Alkylengruppe mit 1 - 6 C-Atomen in der Kette oder eines Restes

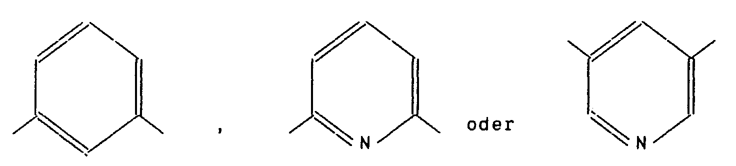

R₁ und R₂ den Rest OH, -O-(C₁-C₄)-Alkyl, -O-(C₃-C₆)-Cycloalkyl, -O-(C₇-C₁₂)Aralkyl oder den Rest NR₃R₄ mit R₃ und R₄ in der Bedeutung Wasserstoff, (C₁-C₄)-Alkyl, (C₃-C₆)-Cycloalkyl, (C₆-C₁₀)-Aryl oder (C₇-C₁₂)-Aralkyl darstellen sowie deren Salze mit physiologisch verträglichen Basen und deren Cyclodextrinclathrate, Verfahren zu Ihrer Herstellung und Ihrer Verwendung als Arzneimittel.

## Beschreibung

Die Erfindung betrifft neue Leukotrien-B₄-Antagonisten, Verfahren zu ihrer Herstellung sowie ihre Verwendung als Arzneimittel.
Leukotrien B₄ (LTB₄) wurde 1979 von B. Samuelsson und Mitarbeiter als Metabolit der Arachidonsäure entdeckt. Bei der Biosynthese wird durch das Enzym 5-Lipoxygenase zunächst als zentrales Zwischenprodukt das Leukotrien A₄ gebildet, das dann durch eine spezifische Hydrolase in das LTB₄ umgewandelt wird.

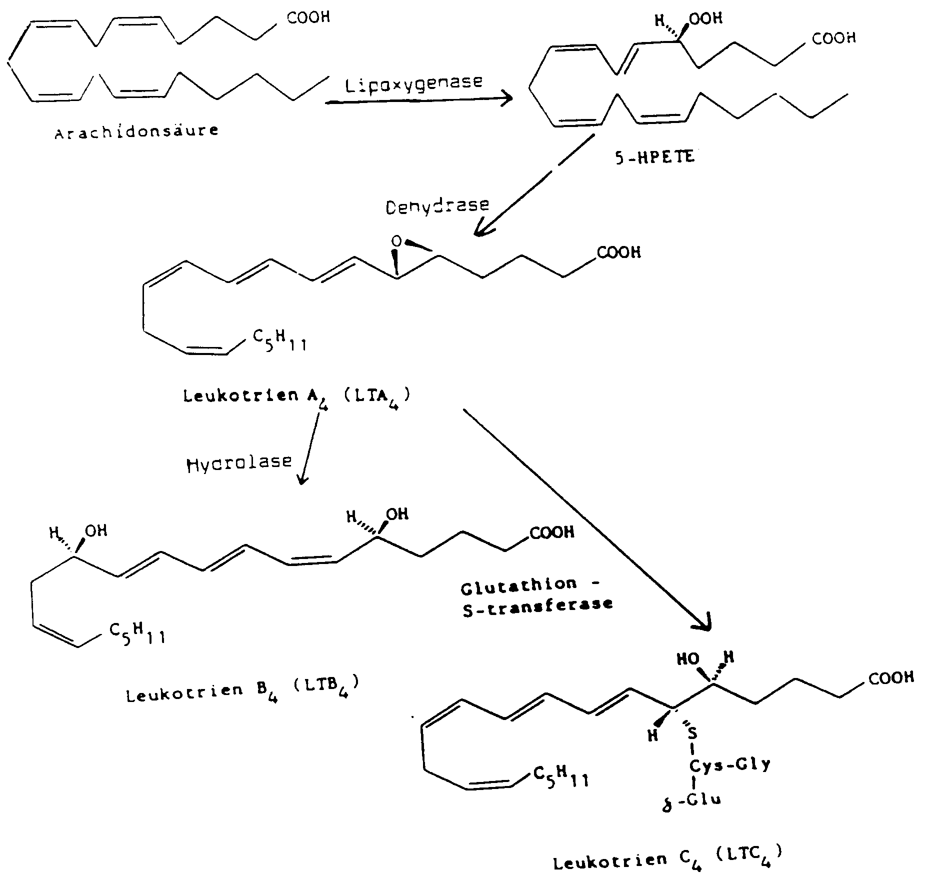

Die Nomenklatur der Leukotriene kann folgenden Arbeiten entnommen werden: a) B. Samuelsson et al., Prostaglandins 19, 645 (1980); 17, 785 (1979). b) C.N. Serhan et al., Prostaglandins 34, 201 (1987).

Die physiologische und insbesondere die pathophysiologische Bedeutung des Leukotrien B₄ wird in einigen neueren Arbeiten zusammengefaßt: a) The Leukotrienes, Chemistry and Biology eds. L.W. Chakrin, D.M. Bailey, Academic Press 1984. b) J.W. Gillard et al., Drugs of the Future 12, 453 (1987). c) B. Samuelsson et al., Science 237, 1171 (1987). d) C.W. Parker, Drug Development Research 10, 277 (1987). Hieraus ergibt sich, daß LTB₄ ein wichtiger Entzündungsmediator für entzündliche Erkrankungen ist, bei denen Leukozyten in das erkrankte Gewebe einwandern.
Von LTB₄ weiß man, daß es die Adhäsion von Leukozyten an die Blutgefäßwand verursacht. LTB₄ ist chemotaktisch wirksam, d.h.es löst eine gerichtete Wanderung von Leukozyten in Richtung eines Gradienten steigender Konzentration aus. Außerdem verändert es aufgrund seiner chemotaktischen Aktivität indirekt die vasculäre Permeabilität, wobei ein Synergismus mit Prostaglandin E₂ beobachtet wird. LTB₄ spielt offensichtlich bei entzündlichen, allergischen und immunologischen Prozessen eine entscheidende Rolle.

Leukotriene und insbesondere LTB₄ sind an Hauterkrankungen beteiligt, die mit entzündlichen Prozessen (erhöhte Gefäßpermeabilität und Ödembildung, Zellinfiltration), erhöhter Proliferation der Hautzellen und Juckreiz einhergehen, wie beispielsweise bei Ekzemen, Erythemen, Psoriasis, Pruritus und Akne. Pathologisch erhöhte Leukotrien-Konzentrationen sind an der Entstehung vieler Dermatitiden entweder ursächlich beteiligt, oder es besteht ein Zusammenhang zwischen der Persistenz der Dermatitiden und den Leukotrienen. Deutlich erhöhte Leukotrienkonzentrationen wurden beispielsweise in der Haut von Patienten mit Psoriasis oder atopischer Dermatitis gemessen.
Weiterhin sind Leukotriene und LTB₄ insbesondere bei Arthritis, chronischer Lungenerkrankungen (z.B. Asthma), Rhinitis und entzündlichen Darmerkrankungen beteiligt.

Antagonisten gegen LTB₄ selbst oder Inhibitoren jener Enzyme, die an der Synthese des LTB₄ beteiligt sind, können als spezifische Heilmittel, besonders gegen Krankheiten, die mit Entzündungen und allergischen Reaktionen einhergehen, wirksam sein.

Aus EP276064 sind bereits Verbindungen mit einer Carboxybenzolphenylpropionsäure-Struktur, die Leukotrien-D₄- und Leukotrien-B₄- antagonistische Eigenschaften besitzen, bekannt.

Es wurden Verbindungen gefunden, die die Wirkung des natürlichen LTB₄ überraschend stark antagonisieren,

Die Erfindung betrifft Leukotrien-B₄-Antagonisten der Formel I, 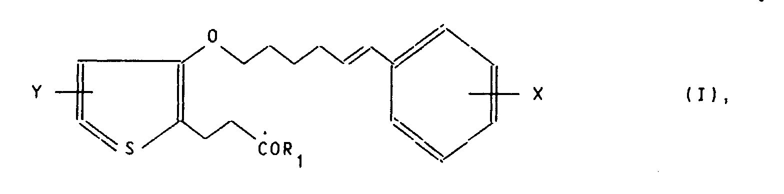
worin
- X: C₁-C₄-Alkoxy oder - S(O)ₚ-(C₁-C₄)-Alkyl,
- p: 0, 1 oder 2
- Y: ein Wasserstoffatom oder den Rest CO-A-COR mit A in der Bedeutung einer Alkylengruppe mit 1 - 6 C-Atomen in der Kette oder eines Restes

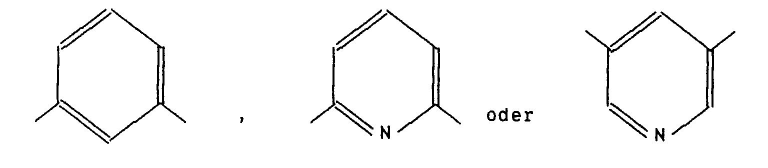

R₁ und R₂ den Rest OH,
-O-(C₁-C₄)-Alkyl, -O-(C₃-C₆)-Cycloalkyl,-O-(C₆-C₁₀)-Aryl, -O-(C₇-C₁₂)-Aralkyl oder den Rest NR₃R₄ mit R₃ in der Bedeutung Wasserstoff, (C₁-C₄)-Alkyl, (C₃-C₆)-Cycloalkyl oder (C₇-C₁₂)-Aralkyl und R₄ in der Bedeutung (C₁-C₄)-Alkyl, (C₃-C₆)-Cycloalkyl oder (C₇-C₁₂)-Aralkyl darstellen sowie deren Salze mit physiologisch verträglichen Basen und deren Cyclodextrinclathrate.

X, R₁ und R₂ als C₁-C₄-Alkoxy-Gruppe können bedeuten: Methoxy, Ethoxy, n-Propoxy, Isoproxy, n-Butoxy, sek.-Butoxy und tert.-Butoxy.

Der C₁-C₄-Alkylrest in der Gruppe -S(O)ₚ-(C₁-C₄)-Alkyl von X als Rest R₃ oder als Rest R₄ kann sein: Methyl, Ethyl, n-Propyl-Isopropyl, n-Butyl, sek.-Butyl, tert.-Butyl.

Als Alkylengruppe Y mit 1-6 C-Atomen kommen geradkettige oder verzweigtkettige, gesättigte Reste in Betracht, wie z. B. Methylen, Ethylen, Trimethylen, Tetramethylen, Hexamethylen, 1-Methyltrimethylen, 1-Methyl-tetramethylen, 1,1-Dimethyl-trimethylen usw.

Der Rest (C₃-C₆)-Cycloalkyl (für R₁, R₂, R₃ und R₄) kann sein: Cyclopropyl, Cyclobutyl, Cyclopentyl oder Cyclohexyl.

Als Reste C₆-C₁₀-Aryl in der Definition von R₁ und R₂ kommen Phenyl, 1-Naphthyl, 2-Naphthyl in Betracht.

Die Reste C₇-C₁₂-Aralkyl in den Definitionen von R₁, R₂, R₃ und R₄ schließlich stellen die folgenden Gruppen dar: Benzyl, Phenethyl, 3-Phenylpropyl, 4-Phenylbutyl, 1-Methyl-3-phenylpropyl, 1-Methyl-2-phenyl-ethyl usw.

Zur Salzbildung sind anorganische und organische Basen geeignet, wie sie dem Fachmann zur Bildung physiologisch verträglicher Salze bekannt sind. Beispielsweise seien genannt Alkalihydroxide, wie Natrium- und Kaliumhydroxid, Erdalkalihydoxide, wie Calciumhydroxid, Amoniak, Amine, wie Ethanolamin, Diethanolamin, Triethanolamin, N-Methylglucamin, Morphin, Tris-(hydroxymethyl)-methylamin usw.

Um zu den Cyclodextrinclathraten zu gelangen, werden die Verbindungen der Formel I mit α-, β- oder γ-Cyclodextrin umgesetzt. Bevorzugt sind die β-Cyclodextrinclathrate.

Die Erfindung betrifft außerdem ein Verfahren zur Herstellung von Leukotrien-β₄-Antagonisten der Formel I, dadurch gekennzeichnet, daß man in an sich bekannter Weise eine Verbindung der Formel II, 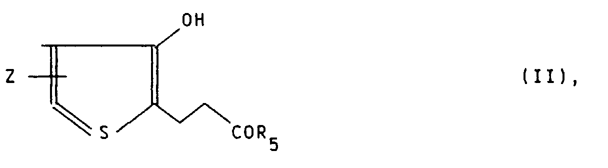
worin Y die oben angegenene Bedeutung hat und R₅ die Reste OH oder CO-CH₂-COOR₆ bedeutet, wobei R₆ eine C₁-C₄-Alkylgruppe darstellt, mit einer Verbindung der Formel III, 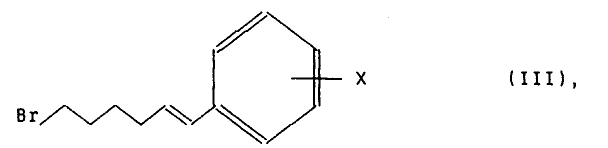
worin X die oben angegebene Bedeutung hat, in Gegenwart von Cäsium-, Lithium- oder Kaliumcarbonat umsetzt und gegebenenfalls die Estergruppen verseift, Carboxylgruppen verestert oder die erhaltenen Säuren der Formel I mit organischen oder anorganischen Basen oder Cyclodextrinen umsetzt.

Das oben genannte Verfahren (II+III=>I) wird in organischen Lösungsmitteln, wie z. B. Dimethylformamid, bei Temperaturen zwischen 0 und 100°C, vorzugsweise bei Temperaturen zwischen 20 und 60°C unter Rühren im Verlaufe von 5-24 Stunden in Gegenwart von Cäsium-, Lithium-oder Kaliumcarbonat durchgeführt.

Die Reduktion der Carbonylgruppe A erfolgt vorzugsweise mit Natriumborhydrid unter den üblichen Bedingungen. Die erhaltenen Hydroxymethylenverbindungen können gewünschtenfalls in die optischen Antipoden aufgetrennt werden.

Die Herstellung der für diese Umsetzung benötigten Verbindungen der Formel II und III erfolgt nach den in den Beispielen, bzw. in den Referenzbeispielen angegebenen Verfahren.

Die Verseifung der Ester der Formel I wird nach den dem Fachmann bekannten Methoden durchgeführt, wie beispielsweise mit basischen Katalysatoren.

Die Einführung der Estergruppe 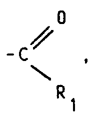
bei welcher R₁ eine 0-Alkylgruppe mit 1-4 C-Atomen darstellt, erfolgt nach den dem Fachmann bekannten Methoden. Die 1-Carboxy-verbindungen werden beispielsweise mit Diazokohlenwasserstoffen in an sich bekannter Weise umgesetzt. Die Veresterung mit Diazokohlenwasserstoffen erfolgt z. B. dadurch, daß man eine Lösung des Diazokohlenwasserstoffes in einem inerten Lösungsmittel, vorzugsweise in Diethylether, mit der 1-Carboxyverbindung in dem gleichen oder in einem anderen inerten Lösungsmittel, wie z. B. Methylenchlorid, vermischt. Nach beendeter Umsetzung in 1 bis 30 Minuten wird das Lösungsmittel entfernt und der Ester in üblicher Weise gereinigt. Diazoalkane sind entweder bekannt oder können nach bekannten Methoden hergestellt werden [Org. Reactions Bd. 8, Seiten 389 - 394 (1954)].

Die Einführung der Estergruppe 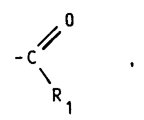
bei welcher R¹ eine -0-Arylgruppe darstellt, erfolgt nach den dem Fachmann bekannten Methoden. Beispielsweise werden die 1-Carboxyverbindungen mit den entsprechenden Arylhydroxyverbindungen mit Dicyclohexylcarbodiimid in Gegenwart einer geeigneten Base, beispielsweise Pyridin, DMAP, Triethylamin, in einem inerten Lösungsmittel umgesetzt. Als Lösungsmittel kommen Methylenchlorid, Ethylenchlorid, Chloroform, Essigester, Tetrahydrofuran, vorzugsweise Chloroform in Frage. Die Reaktion wird bei Temperaturen zwischen -30 °C und +50 °C, vorzugsweise bei 10 °C, durchgeführt.

Die Leukotrien-B₄-Antagonisten der Formel I mit R¹ in der Bedeutung einer COOH-Gruppe können mit geeigneten Mengen der entsprechenden anorganischen Basen unter Neutralisierung in ein Salz überführt werden. Beispielsweise erhält man bein Lösen der entsprechenden Säuren in Wasser, das die stöchiometrische Menge der Base erhält, nach Abdampfen des Wassers oder nach Zugabe eines mit Wasser mischbaren Lösungsmittels, z. B. Alkohol oder Aceton, das feste anorganische Salz.

Zur Herstellung eines Aminsalzes wird die LTB₄-Säure z. B. in einem geeigneten Lösungsmittel, beispielsweise Ethanol, Aceton, Diethylether, Acetonitril oder Benzol gelöst und mindestens die stöchionetrische Menge des Amins dieser Lösung zugesetzt. Dabei fällt das Salz gewöhnlich in fester Form an oder wird nach Verdampfen des Lösungsmittels in üblicher Weise isoliert.

Die Einführung der Amidgruppe 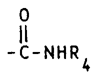
erfolgt nach den dem Fachmann bekannten Methoden. Die Carbonsäuren der Formel I (R₁=OH) werden zunächst in Gegenwart eines tertiären Amins, wie beispielsweise Triethylamin, mit Chlorameisensäureisobutylester in das gemischte Anhydrid überführt. Die Umsetzung des gemischten Anhydrids mit dem Alkalisalz des entsprechenden Amins oder mit Ammoniak erfolgt in einem inerten Lösungsmittel oder Lösungsmittelgemisch, wie beispielsweise Tetrahydrofuran, Dimethoxyethan. Dimethylformamid, Hexamethylphosphorsäuretriamid, bei Temperaturen zwischen 30°C und +60°C, vorzugsweise bei 0°C bis 30°C.

Die als Ausgangsmaterial dienenden Verbindungen der allgemeinen Formel II können beispielsweise hergestellt werden indem man in an sich bekannter Weise 3-Oxy-2-carbomethoxy-thiophen (N. Fiesselmann et. al., Chemische Berichte 87, 841 (1954)) mit Tert.-butyldimethylsilylchlorid silyiert und durch anschließende Reduktion mit Diisobutylaluminiumhydrid in den prikmären Alkohol der Formel IV überführt.

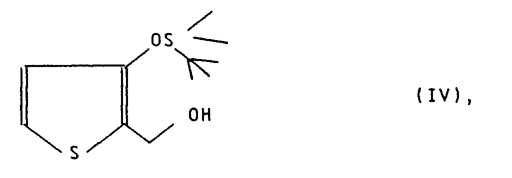

Oxidation der primären Hydroxyxgruppe in IV mit Braunstein zum entsprechenden Aldehyd und Wittig-Horner-Olefinierung mit Phosphonessigsäuretrialkylester sowie anschließende Silylätherspaltung mit Tetrabutylammoniumfluorid, Benzoylierung und Hydrierung des α, β-ungesättigten Esters ergab das Benzoat der Formel V 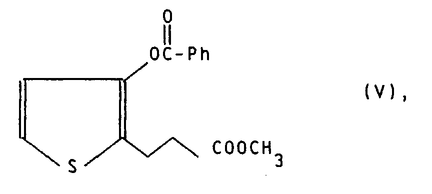
Friedel-Crafts-Acylierung von V und anschließende Benzoatverseifung führte zum Alkohol der allgemeinen Formel II.

Die Verbindungen der Formel I wirken antientzündlich und antiallergisch. Folglich stellen die neuen Leukotrien B₄-Derivate der Formel I wertvolle pharmazeutische Wirkstoffe dar. Die Verbindungen der Formel I sind besonders zur topischen Applikation geeignet, da sie eine Dissoziation zwischen erwünschter topischer Wirksamkeit und unerwünschten systemischen Nebenwirkungen aufweisen.

Die neuen Leukotrien B₄-Antagonisten der Formel I eignen sich in Kombination mit den in der galenischen Pharmazie üblichen Hilfs- und Trägermitteln zur lokalen Behandlung von Kontaktdermatitis, Ekzemen der verschiedensten Art, Neurodermatosen, Erytrodermie, Pruritis vulvae et ani, Rosacea, Erythematodes cutaneus, Psoriasis, Lichen ruber planus et verrucosus und ähnlichen Hauterkrankungen.

Die Herstellung der Arzneimittelspezialitäten erfolgt in üblicher Weise, indem man die Wirkstoffe mit geeigneten Zusätzen in die gewünschte Applikationsform, wie z. B. : Lösungen, Lotionen, Salben, Cremes oder Pflaster, überführt.
In den so formulierten Arzneimitteln ist die Wirkstoffkonzentration von der Applikationsform abhängig. Bei Lotionen und Salben wird vorzugsweise eine Wirkstoffkonzentration von 0,0001 % bis 1 % verwendet.

Darüberhinaus sind die neuen Verbindungen gegebenenfalls in Kombination mit den üblichen Trägermitteln und Hilfsstoffen auch gut zur Herstellung von Inhalationsmitteln geeignet, welche zur Therapie allergischer Erkrankungen der Atemwege wie zum Beispiel des Bronchialasthmas oder der Rhinitis verwendet werden können.

Ferner eignen sich die neuen Leukotrien-B₄-Antagonisten auch in Form von Kapseln, Tabletten oder Dragees, die vorzugsweise 0,1 bis 100 mg Wirkstoff enthalten oder oral appliziert werden oder in Form von Suspensionen, die vorzugsweise 1-200 mg Wirkstoff pro Dosiseinheit enthalten und rektal appliziert werden auch zur Behandlung allergischer Erkrankungen des Darmtraktes, wie der Kolitis ulcerosa und der colitis granulomatosa.

Die neuen Leukotrien-B₄-Derivate können auch in Kombination, wie z.B mit Lipoxygenasehemmern, Cyclooxygenasehemmern, Prostacyclinagonisten, Prostaglandinagonisten, Thromboxanantagonisten, Leukotrien D₄-antagonisten, Leukotrien-E₄-antagonisten, Leukotrien F₄-antagonisten, Phosphodiesterasehemmern, Calciumantagonisten oder PAF-Antagonisten verwendet werden.

Die nachfolgenden Ausführungsbeispiele dienen zur näheren Erläuterung des erfindungsgemäßen Verfahrens.

### Beispiel 1

### 3-{5-(3-Methoxycarbonylbenzoyl)-3-[6-(4-methoxyphenyl)-(5E)-5-hexenyloxy]-2-thienyl}-propionsäuremethylester

Zu einer Lösung von 430 mg 3-[5-(3-Methoxycarbonylbenzoyl)-3-hydroxy-2-thienyl]-propionsäuremethylester und 332 mg (1E)-6-Brom-1-(4-methoxyphenyl)-1-hexen in 2,9 ml Dimethylformamid werden 804 mg Cäsiumcarbonat gegeben und die Suspension 21 Stunden bei Raumtemperatur gerührt. Die Reaktionsmischung wird filtriert, der Filterrückstand mit Dichlormethan gewaschen, das Filtrat eingeengt und der Rückstand an Kieselgel mit Hexan chromatographiert. Es werden 542 mg der Titelverbindung als farbloses Öl erhalten.

IR (CHCl₃) : 2942, 1723, 1670, 1628, 1605, 1435, 1375, 995, 993 cm⁻¹.

Das Ausgangsmaterial für die obige Titelverbindung wird wie folgt hergestellt:

### 1a) 2-Hydroxymethyl-3-(tert.-butyl-dimethylsilyloxyy)-thiophen

Zu einer Lösung von 3 g 3-Oxy-2-carbomethoxy-thiophen (H. Fiesselmann et al., Chemische Berichte 87, 841 (1954)) in 15 ml Dimethylformamid fügt man bei 0 °C 4 g tert.-Butyldimethylsilylchlorid und 3,6 g Imidazol und rührt 22 Stunden bei 22 °C unter Argon. Anschließend verdünnt man mit 500 ml Ether, schüttelt mit 30 ml 10 %ige Schwefelsäure und wäscht mit Wasser neutral. Man trocknet über Magensiumsulfat, dampft im Vakuum ein und chromatographiert den Rückstand an Kieselgel. Mit Hexan/Ether (92+8) eluiert man 5,4 g 2-Carbomethoxy-3-(tert.-butyl-dimethylsilyloxy)-thiophen als farbloses Öl.

Zur Reduktion der Estergruppe löst man 1,1 g des vorstehend hergestellten Silylethers in 25 ml Toluol und tropft bei -70 °C 6,7 ml einer 1,2 molaren Lösung von Diisobutylaluminiumhydrid in Toluol zu. Man rührt 25 Minuten bei -70 °C, versetzt mit 2 ml Isopropanol, rührt 3 Minuten, fügt 3,3 ml Wasser zu, rührt 2 Stunden bei 22 °C, versetzt mit Essigester, filtriert und dampft im Vakuum ein. Dabei erhält man 1,1 g der Titelverbindung als farbloses Öl.

IR: 3600, 3450, 2960, 2935, 2860, 1605, 1555, 1395, 840, 826 cm⁻¹.

### 1b) 3-((3-Benzoyloxy)-2-thienyl)-propionsäure-methylester

Zu einer Lösung von 490 mg des nach Beispiel 1a hergestellten Alkohols in 3 ml Toluol fügt man innerhalb von 20 Minuten 1,8 g Braunstein und rührt 2,5 Stunden bei 22 °C. Anschließend filtriert man, dampft im Vakuum ein und chromatographiert den Rückstand an Kieselgel. Mit Hexan/Ether (9+1) erhält man 390 mg des Aldehyds als farbloses Öl.

IR: 2960, 2938, 1650, 1530, 1435, 1410, 830 cm⁻¹.

Zur Olefinierungsreaktion tropft man bei 0 °C eine Lösung aus 352 mg Phosphonoessigsäuretrimethylester in 3,7 ml Tetrahydrofuran zu einer Suspension von 78 mg Natriumhydrid (55 %ig in Mineralöl) in 6,8 ml Tetrahydrofuran, setzt 76 mg wasserfreies Lithiumchlorid zu und rührt 10 Minuten bei 0 °C. Anschließend tropft man eine Lösung von 360 mg des vorstehend hergestellten Aldehyds in 2,2 ml Tetrahydrofuran zu, rührt 3 Stunden bei 0 °C und neutralisiert dann mit Essigsäure. Man verdünnt mit Ether, wäscht zweimal mit Wasser, trocknet über Natriumsulfat und dampft im Vakuum ein. Den Rückstand reinigt man durch Chromatographie.

8.8 g des vorstehend hergestellten Benzoats löst man in 80 ml Essigester, fügt 900 mg Palladium (10 %ig auf Kohle) zu und hydriert in einem Autoklaven 3 Stunden bei 22 °C und 10 bar Druck. Anschließend wird filtriert und eingedampft. Der Rückstand wird durch Chromatographie an Kieselgel gereinigt. Mit Hexan/Ether (7+3)erhält man 6,65 g der Titelverbindung als schwach gelb gefärbtes Öl.

IR: 2958, 1736, 1625, 1604, 1263 cm⁻¹.

### 1c) 3-{5-(Methoxycarbonylbenzoyl)-3-hydroxy-2-thienyl}-propionsäuremethylester

Zu einer Lösung von 4 g des in Beispiel 1 b hergestellten Benzoats und 4,1g Isophthalsäuremonomethylesterchlorid in 4,9 ml Nitromethan tropft man bei 0 °C 5,5 ml einer Lösung von Aluminiumchlorid in Nitromethan (Herstellung: Man löst 10 g Aluminiumchlorid in 10 ml Nitromethan) und rührt 20 Minuten bei 0 °C und 2 Stunden bei 22 C. Anschließend gießt man auf 50 ml Eiswasser, schüttelt dreimal mit Ether, wäscht die organische Phase mit Natriumbicarbonatlösung und Wasser, trocknet über Natriumsulfat und dampft im Vakuum ein. Den Rückstand reinigt man durch Chromatographie an Kieselgel. Dabei erhält man mit Ether/Hexan (1+1) 1,8 g des Ketons als farbloses Öl.

IR: 2960, 1736, 1640, 1602, 1260 cm⁻¹.

Zur Benzoatabspaltung fügt man zu einer Lösung von 1,1 g des vorstehend hergestellten Ketons in 22 ml Methanol 168 mg wasserfreies Kaliumkarbonat und rührt 2,5 Stunden bei 22 °C unter Argon. Anschließend säuert man mit einer 5 %igen Schwefelsäure an, verdünnt mit Ether, wäscht mit Sole neutral, trocknet über Magnesiumsulfat und dampft im Vakuum ein. Den Rückstand chromatographiert man an Kieselgel. Dabei erhält man mit Ether/Hexan (1+1) 0,7 g der Titelverbindung als gelbliches Öl.

IR (KBr) : 3420 (breit), 3180, 2960, 1740, 1725, 1610 cm⁻¹.

### Beispiel 2

### 3-{5-(3-Carboxybenzoyl)3-[6-(4-methjoxyphenyl)-(5E)-5-hexenyloxy]-2-thienyl}-propionsäure

Zu einer Lösung von 415 mg des in Beispiel 1 hergestellten Diesters in 3,1 ml Methanol fügt man 3,1 ml einer wässrigen 1 normalen Kaliumhydroxydlösung und rührt 4 Stunden bei 22 °C. Anschließend säuert man mit 10 %iger Schwefelsäure auf pH 2 an, extrahiert mit Essigester, wäscht die organische Phase mit Sole, trocknet mit Natriumsulfat und dampft im Vakuum ein. Den Rückstand reinigt man durch Chromatographie an Kieselgel. Mit Essigester/Hexan erhält man 274 mg der Titelverbindung als farblose Kristalle (Schmelzpunkt 133 °C aus Essigester/Hexan umkristallisiert).

IR (KBr) : 3420, 2955, 1700, 1638, 1605, 1510 cm⁻¹.

## Patentansprüche

1. Leukotrien-B₄-Antagonisten der Formel I, 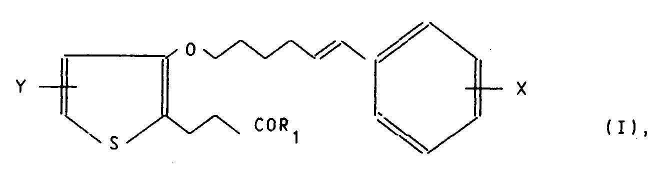 worin
X C₁-C₄-Alkoxy oder S(O)ₚ-(C₁-C₄)-Alkyl,
p 0, 1 oder 2
Y ein Wasserstoffatom oder den Rest COACOR₂ mit A in der Bedeutung einer Alkylengruppe mit 1 - 6 C-Atomen in der Kette oder eines Restes 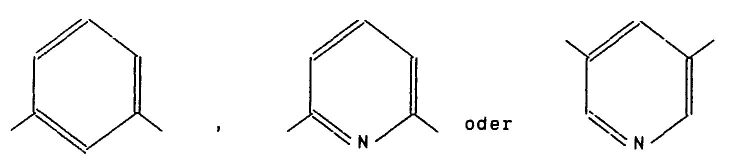
R¹ und R₂ den Rest OH, -O-(C₁-C₄)-Alkyl, -O-(C₃-C₆)-Cycloalkyl, -O-(C₆-C₁₀)Aryl, -O-(C₇-C₁₂)-Aralkyl oder den Rest NR₃R₄ mit R₃ und R₄ in der Bedeutung Wasserstoff, (C₁-C₄)-Alkyl, (C₃-C₆)-Cycloalkyl oder (C₇-C₁₂)-Aralkyl darstellen sowie deren Salze mit physiologisch verträglichen Basen und deren Cyclodextrinclathrate.

2. Verfahren zur Herstellung von Leukotrien-B₄-Antagonisten der Formel I, dadurch gekennzeichnet, daß man in an sich bekannter Weise eine Verbindung der Formel II, 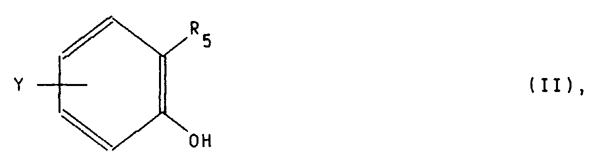 worin Y die oben angegenene Bedeutung hat und R₅ die Reste OH oder COCH₂-COOR₆ bedeutet, wobei R₆ eine C₁-C₄-Alkylgruppe darstellt, mit einer Verbindung der Formel III, 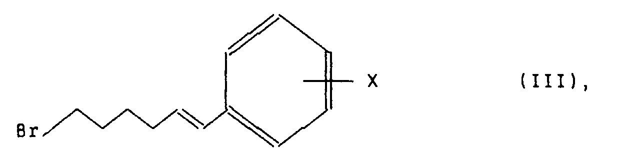 worin Y die oben angegebene Bedeutung hat, in Gegenwart von Cäsium-, Lithium- oder Kaliumcarbonat umsetzt und gegebenenfalls Estergruppen verseift, Carboxylgruppen verestert oder erhaltenen Säuren der Formel I mit organischen oder anorganischen Basen oder Cyclodextrinen umsetzt.

3. Arzneimittel, bestehend aus einer oder mehreren Verbindungen gemäß Anspruch 1 und üblichen Hilfs- und Trägerstoffen.
